# EUROPEAN PATENT APPLICATION

(11) **EP 3 954 283 A1**
(43) Date of publication of application: **16.02.2022**
(21) Application number: 20787611.1
(22) Date of filing: 17.03.2020
(51) Int. Cl.: A61B 5/0444

(54) **FETAL HEART RATE FLUCTUATION MONITORING DEVICE**

(30) Priority: 10.04.2019 JP 2019074960
(71) Applicant: Atom Medical Corporation, Bunkyo-ku Tokyo 113-0033 (JP)
(72) Inventor: SUDO, Kazuhiko, Saitama City, Saitama 338-0835 (JP); ODAGIRI, Naoko, Saitama City, Saitama 338-0835 (JP); OOWADA, Kazunari, Saitama City, Saitama 338-0835 (JP); SONE, Yoshikatsu, Saitama City, Saitama 338-0835 (JP); MATSUBARA, Ichiro, Tokyo 113-0033 (JP)
(74) Representative: DREISS Patentanwälte PartG mbB
(86) International application number: PCT/JP2020/011674
(87) International publication number: WO 2020/209015

(57) **Abstract**

Provided is a fetal heart rate variability monitoring device capable of easily determining a fetal condition. A fetal heart rate variability monitoring device (101) includes a processing unit (11) and a display unit (12) that acquire a fetal heart rate and display a fetal heart rate diagram showing the fetal heart rate over time, an LTV and STV conversion processing unit (35) that calculates STV and LTV from the fetal heart rate, and an event input unit (41) that can designate a predetermined period on the fetal heart rate diagram. The processing unit and the display unit have a function of issuing notification of the STV and LTV in the predetermined period calculated by the LTV and STV conversion processing unit when the predetermined period is designated by the event input unit.

## Description

### Technical Field

The present invention relates to a fetal heart rate variability monitoring device. Priority is claimed on Japanese Patent Application No. 2019-74960, filed on April 10, 2019, the content of which is incorporated herein by reference.

### Background Art

A heart rate is adjusted by an autonomic nervous system or an endocrine system, with a cardiovascular center of a medulla oblongata changing a firing rate for each heart rate with a firing rate of a sinoatrial node of a heart. Heart rate variability is a simple non-invasive method and is used as an index of an autonomic nervous tone of the heart by measuring variability of the heart rate between R and R' (interval between R waves).

For example, PTL 1 describes a delivery monitoring device that records a fetal heart rate curve indicating a change in a fetal heart rate (FHR) over time and a maternal labor pain curve indicating a change in a labor pain intensity (uterine contraction pressure) over time on recording sheet. A display unit in this delivery monitoring device is provided with a display unit relating to transient bradycardia (early, delayed, variant, and prolonged), a display unit relating to heart rate baseline fine variability (disappearance, decrease, moderate, and increase), and a warning sign, together with fetal heart rate display unit that digitally displays (numerically displays) the fetal heart rate, a labor pain intensity display unit that digitally displays (numerically displays) the labor pain intensity, and a fetal heart rate baseline display unit that digitally displays (numerical displays) a fetal heart rate baseline. As described above, PTL 1 describes that main information, such as the fetal heart rate baseline, and a warning, such as urgency, are displayed on the display unit of the delivery monitoring device. Therefore, a high level of interpretation work for determining a health state of a fetus is unnecessary and a burden on a doctor is reduced.

As a method of detecting the fetal heart rate in such a delivery monitoring device in the related art, an internal measurement method in which an electrocardiogram electrode is directly attached to the fetus after membrane rupture to directly detect an electrocardiogram of the fetus and an external measurement method (ultrasonic Doppler method) in which the movement of the fetal heart is indirectly detected by an ultrasonic transducer or the like, which is attached to a maternal abdominal wall are used. The internal measurement method, of the above methods, cannot be used before the membrane rupture since the electrocardiogram electrode is directly attached to the fetus after the membrane rupture (at the time of delivery). The ultrasonic Doppler method using the ultrasonic transducer often contains noise from the maternal body, and it is difficult to appropriately detect the fetal heart rate.

PTL 2 discloses an electrocardiogram signal processing method and an electrocardiogram signal processing device for extracting a fetal electrocardiogram signal (signal corresponding to fetal bioelectric signal) included in a biopotential signal (abdominal electrocardiogram signal) detected from the electrode attached to the maternal body. As described in PTL 2, the usefulness of the fetal bioelectric signal is widely recognized, and a method of extracting the fetal bioelectric signal not only during the delivery but also by a non-invasive method is considered. As described above, when the fetal bioelectric signal can be extracted, it is considered that the heart rate between R and R' (interval between R waves) can be accurately detected and the variability of the fetal heart rate can be accurately grasped with beat-to-beat.

### Citation List

### Patent Literature

[PTL 1] JP-A-2006-223335
[PTL 2] Japanese Patent No. 4590554

### Summary of Invention

### Technical Problem

However, even when the fetal heart rate is calculated based on the fetal bioelectric signal described in PTL 2 and a fetal heart rate diagram is displayed on the display unit, a doctor needs to see a waveform from the fetal heart rate diagram with the naked eye to determine the fetal heart rate variability, and the judgment of the determination may differ depending on the doctor. In this case, as in PTL 1, even when the fetal heart rate baseline fine variability is periodically displayed from the fetal heart rate baseline within a predetermined range, the fetal heart rate variability may not be appropriately evaluated.

The present invention has been made in view of such circumstances, and an object of the present invention is to provide a fetal heart rate variability monitoring device capable of appropriately evaluating fetal heart rate variability and appropriately determining a fetal condition.

### Solution to Problem

A fetal heart rate variability monitoring device of the present invention includes display means for acquiring a fetal heart rate and displaying a fetal heart rate diagram showing the fetal heart rate over time, calculation means for calculating variability information which is information about fetal heart rate variability from the fetal heart rate, a designated period input unit capable of designating a predetermined period on the fetal heart rate diagram, and notification means for issuing notification of the variability information in the predetermined period calculated by the calculation means when the predetermined period is designated by the designated period input unit.

In the present invention, the user designates the predetermined period in which the variability information in the fetal heart rate diagram is desired to be recognized, and the variability information in the predetermined period is notified. Therefore, it is possible to appropriately evaluate the fetal heart rate variability and thus easily determine a fetal condition in the predetermined period.

The variability information includes variability of the fetal heart rate (including amplitude variability and frequency variability over time of fetal heart rate curve), a value indicating the fetal heart rate baseline fine variability (STV or LTV), and changing of the display color of the value according to the value, changing of the alarm sound according to the value, or the like. That is, the above notification is not limited to the display and includes sound output.

The Short Term Variability (STV) is a change having a fastest changing speed and a high frequency among pieces of fetal heart rate baseline fine variability. The STV generally means a change for each fetal heart rate interval time or for each heart rate of an instantaneous fetal heart rate. The Long Term Variability (LTV) has a slower change and a lower frequency than the STV and thus can be visually recognized on the fetal heart rate diagram. The LTV usually means a relatively gradual fetal heart rate baseline fine variability of 2 to 6 times per minute.

As a preferred aspect of the fetal heart rate variability monitoring device of the present invention, the calculation means may calculate at least one of values of STV and LTV at a time interval set in advance, and the display means may display the value, which is successively calculated, in a graph.

In the above aspect, at least one of the values of STV and LTV is calculated at the time interval set in advance and at least one of STV and LTV is successively displayed in a graph. Therefore, it is possible to recognize the change of at least one of STV and LTV over time and thus easily determine a fetal condition.

Since the predetermined period can be designated after recognizing the change of at least one of STV and LTV over time, it is possible to determine the fetal condition more easily. Specifically, in a case where the user has doubts about the STV or LTV value calculated and displayed at time interval set in advance, the user sets the predetermined period excluding a questionable area (for example, area where noise occurs in fetal heart rate). Therefore, it is possible to acquire the appropriate variability information and determine a fetal condition more easily.

As a preferred aspect of the fetal heart rate variability monitoring device of the present invention, the notification means may cause the display means to display information indicating the predetermined period designated by the designated period input unit in correspondence with the fetal heart rate diagram.

In the above aspect, since the information indicating the predetermined period is displayed corresponding to the fetal heart rate diagram, the predetermined period in the fetal heart rate diagram becomes clear.

As a preferred aspect of the fetal heart rate variability monitoring device of the present invention, when the predetermined period is designated by the designated period input unit, the notification means may cause the display means to display the variability information in the predetermined period together with the information indicating the predetermined period near the fetal heart rate diagram.

In the above aspect, since the variability information in the predetermined period can be visually recognized, it is possible to determine the fetal condition more easily.

As a preferred aspect of the fetal heart rate variability monitoring device of the present invention, the notification means may display at least one of the variability information and the information indicating the predetermined period in a different aspect according to a value of the variability information.

In the above aspect, at least one of the variability information and the information indicating the predetermined period is displayed in the different aspect according to the value of the variability information. Therefore, the user can recognize the urgency of the value of the variability information according to the display aspect, and thus it is possible to reliably determine the fetal condition.

As a preferred aspect of the fetal heart rate variability monitoring device of the present invention, the notification means may display the information indicating the predetermined period as a bar extending according to the predetermined period along a time axis of the fetal heart rate diagram.

In the above aspect, the information indicating the predetermined period is displayed as the bar extending along the time axis of the fetal heart rate diagram. Therefore, the predetermined period in the fetal heart rate diagram becomes clearer.

As a preferred aspect of the fetal heart rate variability monitoring device of the present invention, the variability information may include the information indicating the variability of the fetal heart rate, the notification means may display an area including a fetal heart rate curve in the predetermined period of the fetal heart rate diagram, and a size of the area in a vertical axis direction of the fetal heart rate diagram may be set according to the variability information.

In the above aspect, the size of the area including the fetal heart rate curve in the predetermined period is set according to the variability information. Therefore, the user can recognize the size of the fetal heart rate variability simply by visually recognizing the size of the area, and thus it is possible to determine a fetal condition more appropriately.

As a preferred aspect of the fetal heart rate variability monitoring device of the present invention, the notification means may display a frame surrounding the fetal heart rate curve in the predetermined period of the fetal heart rate diagram, and a height of the frame may be set according to a height from a minimum value to a maximum value of the fetal heart rate in the fetal heart rate curve in the predetermined period.

In the above aspect, the height of the frame is larger as the difference between the maximum value and the minimum value of the fetal heart rate in the predetermined period is larger. Therefore, the user can recognize that the fetal heart rate variability is large simply by visually recognizing the size of the frame displayed in the fetal heart rate diagram and thus it is possible to determine the fetal condition more appropriately.

As a preferred aspect of the fetal heart rate variability monitoring device of the present invention, the display means may display an icon for setting the predetermined period, and when the icon and a predetermined position on the fetal heart rate diagram is selected, the designated period input unit may designate the predetermined period with the predetermined position as a start point of the predetermined period.

In the above aspect, when the icon of the predetermined period and the predetermined position on the fetal heart rate diagram are selected, the start point of the predetermined period is set to the predetermined position. Therefore, it is possible to easily set the predetermined period.

As a preferred aspect of the fetal heart rate variability monitoring device of the present invention, the display means may display the icon outside the fetal heart rate diagram, and when a movement operation from a display position of the icon to a predetermined position on the fetal heart rate diagram is detected, the designated period input unit may designate the predetermined period with an end position of the movement operation as the start point of the predetermined period.

In the above aspect, the predetermined period can be set simply by moving (for example, dragging & dropping) the icon. Therefore, it is possible to set the predetermined period more easily. The end position of the movement operation (for example, drop position) is the start point of the predetermined period. Therefore, the position of the start point of the predetermined period can be considered while moving the icon on the fetal heart rate diagram, and thus it is possible to set the predetermined period more appropriately.

### Advantageous Effects of Invention

According to the present invention, an index capable of determining the fetal heart rate variability within a period desired by the user is displayed together with the fetal heart rate (fetal heart rate diagram), and thus it is possible to easily determine a fetal condition.

### Brief Description of Drawings

Fig. 1 is a block diagram describing a configuration of a fetal heart rate variability monitoring device according to an embodiment of the present invention.
Fig. 2 is a block diagram describing a configuration of a processing unit of the fetal heart rate variability monitoring device shown in Fig. 1.
Fig. 3 is a front view of a display unit of the fetal heart rate variability monitoring device and is a diagram showing a display example during measurement of information such as a fetal heart rate.
Fig. 4 is a front view of a display unit of a fetal heart rate variability monitoring device according to a first modification example of the above embodiment.
Fig. 5 is a front view of a display unit of a fetal heart rate variability monitoring device according to a second modification example of the above embodiment.
Fig. 6 is a front view of a display unit of a fetal heart rate variability monitoring device according to a third modification example of the above embodiment.
Fig. 7 is a front view of a display unit of a fetal heart rate variability monitoring device according to a fourth modification example of the above embodiment.
Fig. 8 is a front view of a display unit of a fetal heart rate variability monitoring device according to a fifth modification example of the above embodiment.
Fig. 9 is a front view of a display unit of a fetal heart rate variability monitoring device according to a sixth modification example of the above embodiment. Description of Embodiments

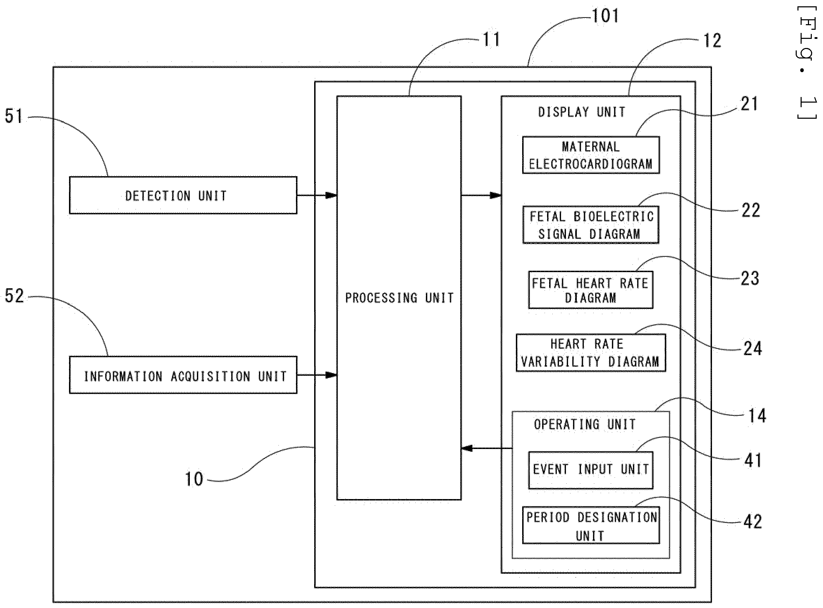

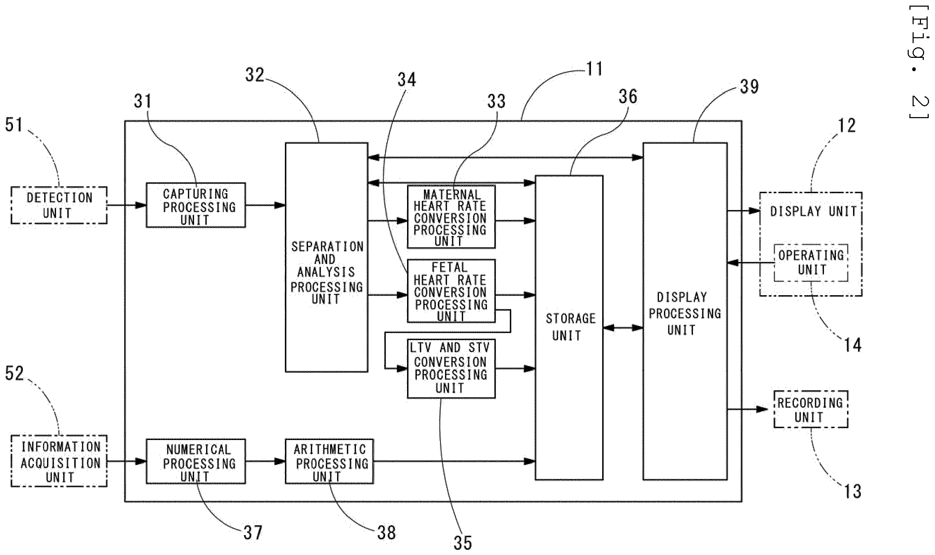

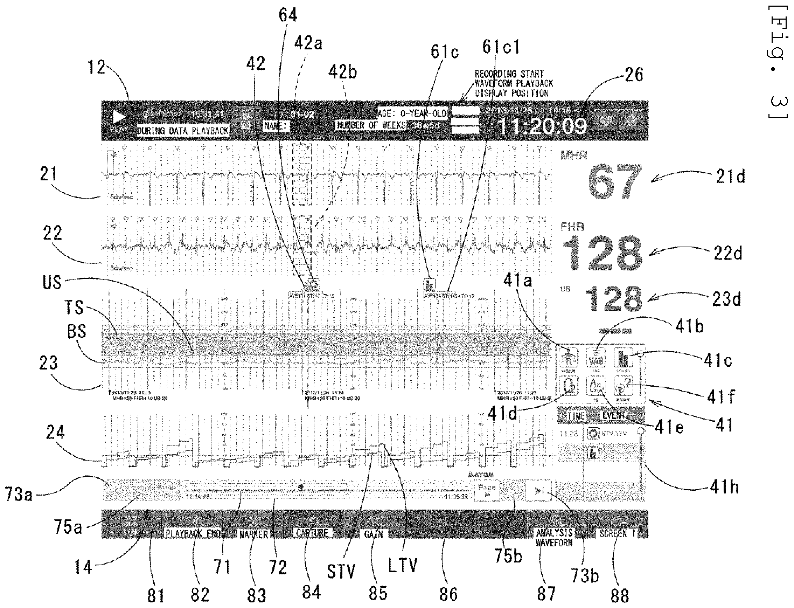

Hereinafter, embodiments of a fetal heart rate variability monitoring device of the present invention will be described with reference to drawings. Fig. 1 is a block diagram describing a configuration of a fetal heart rate variability monitoring device 101 (hereinafter, may be referred to as monitoring device 101) of the present embodiment. Fig. 2 is a block diagram describing a configuration of a processing unit 11 of the monitoring device 101. Fig. 3 is a front view (view of display screen) of a display unit 12 of the monitoring device 101.

### [Overall Configuration of Fetal Heart Rate Variability Monitoring Device]

As shown in the overall block diagram of Fig. 1, the monitoring device 101 of the present embodiment includes a device main body 10, a detection unit 51 connected to the device main body 10, and an information acquisition unit 52 that acquires information transmitted from a delivery monitoring device (not shown). The delivery monitoring device is provided with a labor pain transducer, an ultrasonic transducer, or the like (both not shown), which is attached to an abdomen of a maternal body.

As shown in the block diagram of Fig. 1, the device main body 10 includes a processing unit 11 including a computer that arithmetically processes signals acquired from the detection unit 51 and the information acquisition unit 52, and the display unit 12 (refer to Fig. 3) that displays various types of information about the maternal body and a fetus obtained by the processing unit 11.

The detection unit 51 connected to the device main body 10 is a sensor for detecting four pieces of information of a maternal electrocardiogram signal, a fetal bioelectric signal, a maternal heart rate, and a fetal heart rate. Although not shown, the detection unit 51 is composed of a plurality of abdominal electrodes and a plurality of chest electrodes, each of which is attached to the maternal body. The abdominal electrode attached to the abdomen of the maternal body detects a biopotential signal in which various signals, such as the maternal electrocardiogram signal generated from a maternal heart, a uterine electromyogram signal, a maternal electromyogram signal, and the fetal bioelectric signal generated from a fetal heart of the fetus in the maternal body, are combined. The chest electrode attached to a chest of the maternal body detects the maternal electrocardiogram signal that does not include the fetal bioelectric signal.

On the other hand, the labor pain transducer connected to the delivery monitoring device is, for example, a pressure-sensitive sensor that detects a pressure increase caused by expansion and compression of the abdomen of the maternal body during uterine contraction. Similarly, the ultrasonic transducer connected to the delivery monitoring device is, for example, an ultrasonic sensor for detecting a fetal heart beat by an ultrasonic Doppler method. Each of the pressure change detected by the labor pain transducer and the fetal heart beat detected by the ultrasonic transducer is converted into an electric signal and transmitted to the processing unit 11 of the device main body 10 through the information acquisition unit 52.

Each signal detected by the detection unit 51 and a signal input to the information acquisition unit 52 are transmitted to the processing unit 11 of the device main body 10, as shown in the block diagram of Fig. 1. In the processing unit 11, the fetal bioelectric signal is extracted by using these signals. The monitoring device 101 of the present embodiment employs the electrocardiogram signal processing method described in PTL 2 (Japanese Patent No. 4590554), and the fetal bioelectric signal is extracted based on this electrocardiogram signal processing method.

More specifically, as shown in the block diagram of the processing unit 11 of Fig. 2, the processing unit 11 is provided with a capturing processing unit 31, a separation and analysis processing unit 32, a maternal heart rate conversion processing unit 33, a fetal heart rate conversion processing unit 34, an LTV and STV conversion processing unit 35, a storage unit 36, a numerical processing unit 37, an arithmetic processing unit 38, and a display processing unit 39. The signal detected by the detection unit 51 is transmitted to the capturing processing unit 31 and is converted into data suitable for analysis by the separation and analysis processing unit 32 in the capturing processing unit 31. For the data conversion processing performed by the capturing processing unit 31, specifically, signal processing such as signal amplification, AD conversion, or data section division for recording and storage processing is performed.

The signal processed by the capturing processing unit 31 is passed from the capturing processing unit 31 to the separation and analysis processing unit 32. The separation and analysis processing unit 32 performs electrocardiogram signal processing, and the fetal bioelectric signal is extracted from the biopotential signal passed from the capturing processing unit 31. The fetal bioelectric signal extracted by the separation and analysis processing unit 32 is passed from the separation and analysis processing unit 32 to the fetal heart rate conversion processing unit 34. The fetal heart rate conversion processing unit 34 calculates the fetal heart rate from an interval change between signals corresponding to R waves obtained from the fetal bioelectric signal. The maternal electrocardiogram signal detected by the chest electrode of the detection unit 51 is passed from the separation and analysis processing unit 32 to the maternal heart rate conversion processing unit 33. In the maternal heart rate conversion processing unit 33, the maternal heart rate is calculated from an interval change between R waves obtained from the maternal electrocardiogram signal. The fetal bioelectric signal, the fetal heart rate, the maternal electrocardiogram signal, and the maternal heart rate are recorded in the storage unit 36.

A range of increase and decrease in heart rate represented by so-called rhythm disturbance is approximately constant within an individual. When a heart rate variability speed for noise, which tends to have a faster value that normal, is acquired, a significantly faster value is obtained. For this reason, when the heart rate of the maternal body or the fetus with the rhythm disturbance is simply calculated from the fetal bioelectric signal, an unreliable fetal heart rate may be obtained. When a threshold value is simply set to remove the noise, a normal value is also removed. Therefore, an unreliable fetal heart rate may be obtained in this case as well and it is difficult to recognize fetal abnormality.

Therefore, in the present embodiment, the separation and analysis processing unit 32 has a function of detecting variability of the fetal bioelectric signal, fetal heart rate, or the like, a function of detecting arrhythmia, or the like, a function of detecting an analysis error, and the like, based on the information stored in the storage unit 36 in advance.

As described above, in the monitoring device 101 of the present embodiment, the fetal bioelectric signal, the fetal heart rate, the maternal electrocardiogram signal, and the maternal heart rate are acquired by the detection unit 51, the capturing processing unit 31, separation and analysis processing unit 32, maternal heart rate conversion processing unit 33, and fetal heart rate conversion processing unit 34 of the processing unit 11.

In the monitoring device 101 of the present embodiment, the STV and the LTV are detected from the fetal heart rate, acquired by the fetal heart rate conversion processing unit 34, by the LTV and STV conversion processing unit 35 as variability information about the variability of the fetal heart rate. Calculation means in the present invention is configured by the LTV and STV conversion processing unit 35.

The STV in the present embodiment is a change having a fastest changing speed and a high frequency among pieces of fetal heart rate baseline fine variability. The STV generally means a change for each fetal heart rate interval time or for each heart rate of an instantaneous fetal heart rate. The LTV has a slower change and a lower frequency than the STV and thus can be visually recognized on a fetal heart rate diagram. The LTV usually means a relatively gradual fetal heart rate baseline fine variability of 2 to 6 times per minute.

Specifically, in a case where the fetal heart rate (FHR calculated value) calculated during a calculation period (for example, every 10 heart rates) changes exceeding a condition set in advance, the LTV and STV conversion processing unit 35 adds a difference between an STV calculated value and an LTV calculated value of a previously acquired heart rate and an STV calculated value and an LTV calculated value of a current heart rate each time to obtain the STV and the LTV in the calculation period, as shown in Tables 1 and 2.

**[Table 1]**

| Heart rate | FHR calculated value | Change direction | Integrated state | STV integrated value |
|---|---|---|---|---|
| 1 | 120 | - | Reset | 0 |
| 2 | 124 | Up | Hold | 0 |
| 3 | 130 | Up | Hold | 0 |
| 4 | 140 | Up | Hold | 0 |
| 5 | 135 | Down | +5 | 5 |
| 6 | 100 | Hold | Hold | 5 |
| 7 | 133 | Down | Hold | 5 |
| 8 | 129 | Down | Hold | 5 |
| 9 | 133 | Up | +4 | 9 |
| 10 | 142 | Up | Hold | 9 |
| 11 | 128 | Down | Reset | 0 |
| 12 | 132 | Up | +4 | 4 |
| 13 | 128 | Down | +4 | 8 |
| 14 | 135 | Up | +7 | 15 |
| 15 | 110 | Down | +25 | 40 |
| 16 | 95 | Down | Hold | 40 |
| 17 | 138 | Hold | Hold | 40 |

**[Table 2]**

| Heart rate | FHR calculated value | Change direction | Integrated state | LTV integrated value |
|---|---|---|---|---|
| 1 | 120 | - | Reset | 0 |
| 2 | 124 | Up | Hold | 0 |
| 3 | 130 | Up | +6 | 6 |
| 4 | 140 | Up | +10 | 16 |
| 5 | 135 | Down | Hold | 16 |
| 6 | 100 | Hold | Hold | 16 |
| 7 | 133 | Down | +2 | 18 |
| 8 | 129 | Down | +4 | 22 |
| 9 | 133 | Up | Hold | 22 |
| 10 | 142 | Up | +9 | 31 |
| 11 | 128 | Down | Reset | 0 |
| 12 | 132 | Up | Hold | 0 |
| 13 | 128 | Down | Hold | 0 |
| 14 | 135 | Up | Hold | 0 |
| 15 | 110 | Down | Hold | 0 |
| 16 | 95 | Down | +15 | 15 |
| 17 | 138 | Hold | Hold | 15 |

Table 1 shows a method of calculating the STV (STV integrated value). In a case where a current FHR calculated value changes in the same direction as a previously acquired FHR calculated value (for example, as shown in heart rates 2 and 3 in Table 1, in a case where an FHR calculated value of the heart rate 3 corresponding to the "current FHR calculated value" increases from 124 to 130 while a change direction of the heart rate 2 corresponding to the "previously acquired FHR calculated value" is "Up" meaning an increase), the LTV and STV conversion processing unit 35 does not add an absolute value (+6) of the above difference to the STV integrated value. On the other hand, in a case where the current FHR calculated value changes in a direction different from the previously acquired FHR calculated value (for example, as shown in heart rates 4 and 5 in Table 1, in a case where an FHR calculated value of the heart rate 5 corresponding to the "current FHR calculated value" decreases from 140 to 135 while the change direction of the heart rate 4 corresponding to the "previously acquired FHR calculated value" is "Up" meaning an increase), an absolute value (+5) of the above difference is added to the STV integrated value. In a case where the difference between the current FHR calculated value and the previously acquired FHR calculated value is a predetermined amount (for example, ±26 bpm) or more (for example, as shown in heart rates 5 and 6 in Table 1, in a case where the FHR calculated value of the heart rate 5 corresponding to the "previously acquired FHR calculated value" is 135, an FHR calculated value of the heart rate 6 corresponding to the "current FHR calculated value" is 100, and the difference is -35), an absolute value of the above difference is not added to the STV integrated value and the previous FHR calculated value is not updated. More specifically, in calculating the STV integrated value of a heart rate 7 shown in Table 1, an FHR calculated value of the heart rate 7 (133) is compared with the FHR calculated value of the heart rate 5 (135), instead of the FHR calculated value of the heart rate 6 (100). In this case, since the FHR calculated value of the heart rate 7 decreases from 135 to 133 while the change direction of the heart rate 5 is "Down" meaning a decrease, an absolute value (+2) of the above difference is not added to the STV integrated value.

Table 2 shows a method of calculating the LTV (LTV integrated value). In a case where a current FHR calculated value changes in the same direction as a previously acquired FHR calculated value (for example, as shown in heart rates 2 and 3 in Table 2, in the case where the FHR calculated value of the heart rate 3 corresponding to the "current FHR calculated value" increases from 124 to 130 while the change direction of the heart rate 2 corresponding to the "previously acquired FHR calculated value" is "Up" meaning an increase), the LTV and STV conversion processing unit 35 adds the absolute value (+6) of the above difference to the LTV integrated value. On the other hand, in a case where the current FHR calculated value changes in a direction different from the previously acquired FHR calculated value (for example, as shown in heart rates 4 and 5 in Table 2, in the case where the FHR calculated value of the heart rate 5 corresponding to the "current FHR calculated value" decreases from 140 to 135 while the change direction of the heart rate 4 corresponding to the "previously acquired FHR calculated value" is "Up" meaning an increase), the absolute value (+5) of the above difference is not added to the LTV integrated value. In a case where the difference between the current FHR calculated value and the previously acquired FHR calculated value is a predetermined amount (for example, ±26 bpm) or more (for example, as shown in heart rates 5 and 6 in Table 2, in the case where the FHR calculated value of the heart rate 5 corresponding to the "previously acquired FHR calculated value" is 135, the FHR calculated value of the heart rate 6 corresponding to the "current FHR calculated value" is 100, and the difference is -35), the absolute value of the above difference is not added to the LTV integrated value and the previous FHR calculated value is not updated. More specifically, in calculating the LTV integrated value of a heart rate 7 shown in Table 2, the FHR calculated value of the heart rate 7 (133) is compared with the FHR calculated value of the heart rate 5 (135), instead of the FHR calculated value of the heart rate 6 (100). In this case, since the FHR calculated value of the heart rate 7 decreases from 135 to 133 while the change direction of the heart rate 5 is "Down" meaning a decrease, the absolute value (+2) of the above difference is added to the LTV integrated value.

Since the calculation period of the STV and the LTV of the present embodiment is every 10 heart rates, the STV integrated value and the LTV integrated value are reset every 10 heart rates as shown in heart rates 1 and 11 of Tables 1 and 2.

The calculation period of the STV and the LTV may be calculated for each predetermined time (for example, every minute) without using the heart rate as a reference. In this case, since the STV integrated value and the LTV integrated value are reset for each predetermined time, the STV and the LTV are calculated for each predetermined time (for example, every minute).

The STV and the LTV for the calculation period (every 10 heart rates or every minute in the present embodiment) calculated by such a method are stored in the storage unit 36.

In the present embodiment, the STV and the LTV are calculated by the above method, but the present invention is not limited thereto. For example, the STV and the LTV may be calculated from a definition formula shown in Table 1 on page 22 of "On the identity of several variability indices for fetal heart beats" in "Medical Electronics and Biological Engineering" Vol. 23, No. 1 (February 1985).

A signal related to the pressure change detected by the labor pain transducer among signals input to the information acquisition unit 52 from the delivery monitoring device (not shown) is subjected to the signal processing, such as signal amplification, AD conversion, or data section division for recording and storage processing, in the numerical processing unit 37, as shown in the block diagram of the processing unit 11 shown in Fig. 2. The signal processed by the numerical processing unit 37 is passed from the numerical processing unit 37 to the arithmetic processing unit 38, and the labor pain intensity corresponding to 0 to 100 is calculated by the arithmetic processing unit 38. The calculated labor pain intensity is recorded in the storage unit 36.

A signal related to the fetal heart beat detected by the ultrasonic transducer among the signals input to the information acquisition unit 52 from the delivery monitoring device (not shown) is also subjected to the signal processing, such as signal amplification, AD conversion, or data section division for recording and storage processing, in the numerical processing unit 37, and then the fetal heart rate is calculated by the arithmetic processing unit 38 and is stored in the storage unit 36.

The fetal heart rate obtained through the information acquisition unit 52 is different from the fetal heart rate obtained through the detection unit 51 (fetal heart rate calculated by fetal heart rate conversion processing unit 34).

In the present embodiment, the signal input from the delivery monitoring device to the information acquisition unit 52 is subjected to the signal processing by the numerical processing unit 37, and the labor pain intensity and the fetal heart rate are calculated by the arithmetic processing unit 38, but the present invention is not limited thereto. For example, an external device such as a delivery monitoring device may include a processing unit having the same functions as the numerical processing unit 37 and the arithmetic processing unit 38, and information about the labor pain intensity and the fetal heart rate calculated and obtained in the external device may be input to the information acquisition unit 52 of the monitoring device 101. In this case, the processing unit 11 of the monitoring device 101 may not include the numerical processing unit 37 and the arithmetic processing unit 38.

The fetal bioelectric signal, fetal heart rate, maternal electrocardiogram signal, maternal heart rate, STV and LTV, and labor pain intensity recorded in the storage unit 36 are transmitted to the display processing unit 39. In the display processing unit 39, drawing processing is performed based on the above information to create information about a maternal electrocardiogram (maternal electrocardiogram waveform) based on the maternal electrocardiogram signal, a fetal bioelectric signal diagram (fetal bioelectric signal waveform) based on the fetal bioelectric signal, a fetal heart rate diagram based on the fetal heart rate, and a heart rate variability diagram based on the STV and the LTV. As shown in Figs. 1 and 3, the above information is displayed on the display unit 12 as a maternal electrocardiogram 21, a fetal bioelectric signal diagram 22, a fetal heart rate diagram 23, and a heart rate variability diagram 24. The heart rate variability diagram 24 is displayed in the labor pain intensity diagram based on the labor pain intensity and is displayed on a labor pain intensity curve (omitted in Fig. 3) in an overlapped manner.

As described above, in the monitoring device 101 of the present embodiment, with the display processing unit 39 of the processing unit 11 and the display unit 12, information of the STV and the LTV in a predetermined period designated by a user is separately displayed together with information of the maternal electrocardiogram 21, the fetal bioelectric signal diagram 22, the fetal heart rate diagram 23, and the heart rate variability diagram 24. Display means and notification means of the present invention are configured by the display processing unit 39 and the display unit 12.

In the present embodiment, a length of the predetermined period is set to one minute in advance, but the set length (time) can be randomly changed by the user.

The processing method in the detection unit 51 or the processing unit 11 used in the present embodiment is an example. In the present embodiment, a method of measuring and calculating each of the maternal electrocardiogram signal, the fetal bioelectric signal, and the fetal heart rate is not particularly limited. In the present embodiment, the detection unit 51 is used as detection means by an external measurement method. However, detection means by an internal measurement method can be used or detection means different from the present embodiment among pieces of detection means by the external measurement method can be also used. Similarly, for the delivery monitoring device, various types of detection means can be used regardless of the distinction between the internal measurement method and the external measurement method.

In the monitoring device 101 of the present embodiment, the information such as the maternal electrocardiogram signal and the fetal bioelectric signal is acquired by using the signal detected by the detection unit 51, and the maternal heart rate and the fetal heart rate are acquired from the obtained maternal electrocardiogram signal and fetal bioelectric signal. However, a method of acquiring the maternal heart rate or the fetal heart rate is not limited thereto. For example, instead of the detection unit 51, it is possible to use detection means capable of directly acquiring the maternal heart rate and the fetal heart rate by directly attaching the electrode or the like to the maternal body and the fetus.

Next, display contents of the display unit 12 will be described more specifically. As shown in Fig. 3, the display unit 12 is a touch panel type display (monitor) having an input function (operation function) together with a display function of displaying information about the maternal body and the fetus. The maternal electrocardiogram 21, the fetal bioelectric signal diagram 22, the fetal heart rate diagram 23, and the heart rate variability diagram 24 are displayed on a screen of the display unit 12. A waveform of the maternal electrocardiogram signal is displayed on the maternal electrocardiogram 21, a waveform of the fetal bioelectric signal is displayed on the fetal bioelectric signal diagram 22, a fetal heart rate curve TS showing the fetal heart rate over time is displayed on the fetal heart rate diagram 23, and the STV and the LTV are displayed on the heart rate variability diagram 24. A maternal heart rate curve BS and a fetal heart rate curve US showing an externally input fetal heart rate over time are simultaneously displayed on the fetal heart rate diagram 23, in addition to the fetal heart rate curve TS. In the maternal electrocardiogram 21, the fetal bioelectric signal diagram 22, the fetal heart rate diagram 23, and the heart rate variability diagram 24, the horizontal axis is a time axis and the vertical axis is a numerical axis of each index.

A display width (time range) of the horizontal axis in the maternal electrocardiogram 21 and the fetal bioelectric signal diagram 22 is shorter than a display width (time range) of the horizontal axis in the fetal heart rate diagram 23, which is about 1/10 to 1/1,000 (preferably 1/20 to 1/100) of the fetal heart rate diagram 23. Therefore, a display magnification is about 10 times to 1,000 times (preferably 20 times to 100 times). For example, the display width of the fetal heart rate diagram 23 is set to ten and several minutes, while the display width of the maternal electrocardiogram 21 and the fetal bioelectric signal diagram 22 is set to ten and several seconds. Accordingly, the maternal electrocardiogram waveform and the fetal bioelectric signal waveform can be displayed in more detail in the maternal electrocardiogram 21 and the fetal bioelectric signal diagram 22.

As for the information of the fetal heart rate diagram 23 and the heart rate variability diagram 24, a right end (right end on horizontal axis) of Fig. 3 is the latest information. The display screen of the display unit 12 is provided with a recording time display area 26 for displaying a recording time such that the latest time (time at right end of Fig. 3 in the fetal heart rate diagram 23) is displayed.

A fetal heart rate digital display area 22d for digitally displaying the fetal heart rate calculated based on the fetal bioelectric signal detected by the detection unit 51 (fetal heart rate calculated by fetal heart rate conversion processing unit 34) is provided on a right side of the fetal bioelectric signal diagram 22 of the display screen of the display unit 12 such that the waveform of the fetal bioelectric signal and the digitally displayed fetal heart rate can be checked at the same time on one screen of the display unit 12. Similarly, a maternal heart rate digital display area 21d for digitally displaying the maternal heart rate is provided on a right side of the maternal electrocardiogram 21 of the display screen of the display unit 12 such that the waveform of the maternal electrocardiogram signal and the digitally displayed maternal heart rate can be checked at the same time on one screen of the display unit 12. A fetal heart rate digital display area 23d for digitally displaying the fetal heart rate calculated based on the signal input to the information acquisition unit 52 (fetal heart rate calculated by numerical processing unit 37 and arithmetic processing unit 38) is provided on a right side of the fetal heart rate diagram 23 of the display screen of the display unit 12, separately from the fetal heart rate digital display area 22d, such that the fetal heart rate calculated from the fetal bioelectric signal and the externally input fetal heart rate can be checked at the same time. Accordingly, even when a problem such as one of the two fetal heart rates not being digitally displayed temporarily occurs, the user can continue to check the fetal heart rate as long as the other fetal heart rate is digitally displayed.

In addition to the screen shown in Fig. 3, a plurality of screens can be switched and displayed on the display screen of the display unit 12.

Next, the input function (operation function) of the display unit 12 will be specifically described. As shown in Fig. 1, the display unit 12 includes an operating unit 14 having an event input unit 41 and a period designation unit 42. The user operates (for example, presses or touches) an operation switch displayed on the screen of the display unit 12 to perform various operations.

The event input unit 41 includes, for example, event recording buttons 41a to 41f composed of six icons disposed in a lower right portion of the display unit 12 shown in Fig. 3 and a window 41h disposed below the event recording buttons 41a to 41f. The event input unit 41 has functions of receiving an input operation from the user regarding various types of event information, such as changing a position of the maternal body and providing vibration to wake up a sleeping fetus in the maternal body, and of transmitting information about an input when the input is received from the user to the display processing unit 39. The information transmitted to the display processing unit 39 is drawn by the display processing unit 39 and transmitted to the display unit 12 again, or is stored (recorded) in the storage unit 36. In the window 41h, a type of the event recording button input and operated by the user and a time when the event recording button is input and operated are associated with each other and displayed. The type of the event recording button and the operation time are sequentially displayed in a time-series from the upper portion to the lower portion of the window 41h.

The event recording buttons 41a to 41f will be specifically described. For example, the event recording button 41a shown in Fig. 3 is an icon representing a change in the position of the maternal body, the event recording button 41b is an icon representing transmission of a signal providing vibration to wake up the sleeping fetus in the maternal body, the event recording button 41c is an icon representing a marker for disposing the STV and the LTV at a start point of the predetermined period to be calculated, the event recording button 41d is an icon representing oxygen supplying to the maternal body, the event recording button 41e is an icon representing a blood transfusion to the maternal body, and the event recording button 41f is an icon representing a failure of the detection unit 51. At the time of event occurrence, when the user moves (drags) target event recording buttons 41a to 41f to an upper side of the fetal heart rate diagram 23 while pressing the target buttons with a finger, a touch pen, or the like and releases (drops) the finger, the touch pen, or the like at a desired position, the drawing processing is performed by the display processing unit 39, and the event information (for example, simple event recording button 61c shown in Fig. 3) is displayed on the upper side of the fetal heart rate diagram 23.

For example, when the user drags & drops the event recording button 41c on the upper side of the fetal heart rate diagram 23, the simple event recording button 61c is displayed on the upper side of the fetal heart rate diagram 23. The LTV and STV conversion processing unit 35 calculates the LTV and the STV for the predetermined period (for example, one minute) from the start point with a position (point in time) where the simple event recording button 61c is disposed as the start point, and the LTV and the STV are displayed together with an average value of the fetal heart rate in the predetermined period. The detection of the drag & drop operation (movement operation) is executed by the event input unit 41. The event input unit 41 has a function of designating the predetermined period with an end position (that is, second position) of the movement operation as the start point when the movement operation from a position where the event recording button 41c is displayed (first position) to a position on the fetal heart rate diagram 23 (second position) is detected. That is, the event input unit 41 corresponds to a designated period input unit of the present invention.

In the present embodiment, a bar 61c1 extending laterally along the time axis of the fetal heart rate diagram 23 is displayed as information indicating the predetermined period. The bar 61c1 is displayed with a length corresponding to a length of the predetermined period designated by the user. With checking of positions of a start point and an end point of the bar 61c1, the user can clearly recognize the predetermined period on the fetal heart rate diagram 23. Further, the average value of the fetal heart rate, the STV and the LTV in the predetermined period indicated by the bar 61c1 are displayed below the bar 61c1.

In the present embodiment, the case where the display unit 12 is the touch panel type display and the drag & drop operation is performed has been described as an example, but the present invention is not limited thereto. For example, in a case where the display unit is a type of display that displays a pointer on the screen, the target event recording button is selected with the pointer, and the desired position on the fetal heart rate diagram is selected with the pointer. With the selection, it is possible to display the event information (simple event recording button) on the upper side of the fetal heart rate diagram. Even though the display unit is the touch panel type display, when the display unit has a pointer display function, it is possible to display the event information (simple event recording button) on the upper side of the fetal heart rate diagram by, for example, pressing the target event recording button with a finger or a touch pen and then pressing a desired position on the fetal heart rate diagram with the finger or the touch pen.

Further, various selection buttons 81 to 88 are disposed at the lowermost portion of the display unit 12 shown in Fig. 3. The user selects any of these selection buttons 81 to 88 to change the screen displayed on the display unit 12. For example, the selection button 81 is a button for displaying a TOP screen, and the selection button 82 is a button for ending a playback. The selection button 83 is a marker button and can dispose a marker on the fetal heart rate diagram 23 or the like by selecting the selection button 83. The selection button 84 is a capture button and is a so-called screenshot button that converts the screen displayed on the display unit 12 at a timepoint when the selection button 84 is selected into a captured image. In the present embodiment, at the timing when the selection button 84 is selected, a simple event button 64 indicating the capture is displayed on the fetal heart rate diagram 23. The selection button 85 is a button for switching a display height of the waveform, and the selection button 86 is a button used after the screen is switched by the selection button 88. The selection button 87 is an analysis waveform button for displaying an analyzed waveform, and the selection button 88 is a switching button for switching the screen.

Although not shown, in addition to the above event information, in a case where the analysis error or the like occurs in the separation and analysis processing unit 32, the event information indicating the occurrence of the analysis error or the like is automatically displayed on the fetal heart rate diagram 23 of the display unit 12. When the analysis error or the like occurs in the separation and analysis processing unit 32, the information is stored (recorded) in the storage unit 36 and transmitted to the display processing unit 39, subjected to the drawing processing in the display processing unit 39, and then displayed on the fetal heart rate diagram 23 of the display unit 12.

In the heart rate variability diagram 24, the STV and the LTV calculated at a time interval set in advance (for example, one minute interval) are displayed each time the STV and the LTV are calculated. In the present embodiment, the STV and the LTV are displayed in a line graph format in different colors (for example, blue and red) .

The time interval may be stored in the storage unit 36 in advance or may be set by the user.

The display unit 12 can display not only each of the maternal electrocardiogram 21, the fetal bioelectric signal diagram 22, the fetal heart rate diagram 23, and the heart rate variability diagram 24 based on various types of information processed in real time, but also the maternal electrocardiogram 21, the fetal bioelectric signal diagram 22, the fetal heart rate diagram 23, and the heart rate variability diagram 24 based on accumulated various types of information by calling recorded information already accumulated in the storage unit 36.

For example, a scroll bar 71 representing an information accumulation time from a timepoint of a recording start is provided at the lower portion of the fetal heart rate diagram 23 of the display unit 12 shown in Fig. 3. Information within a period surrounded by a scroll frame 72 on the scroll bar 71 is displayed on the fetal heart rate diagram 23 of the display unit 12. As described above, when the information is accumulated exceeding a period that can be displayed on the screen, a part of the information accumulated in the storage unit 36 is displayed on the fetal heart rate diagram 23. Information of a period not displayed on the fetal heart rate diagram 23 on the screen among the information accumulated in the storage unit 36 can be displayed on the fetal heart rate diagram 23 by moving the scroll frame 72 in the left and right direction of the screen along the scroll bar 71.

The scroll frame 72 can be moved to a predetermined position on the scroll bar 71 by tracing the screen of the display unit 12 with a finger. In addition, a period return button 73a and a period feed button 73b disposed on the left and right of the scroll bar 71 are operated (pressed, touched) to move the scroll frame 72 on a period adjacent to a current display period, and information about the fetal heart rate or the LTV and STV in the period can be displayed on the fetal heart rate diagram 23 or the heart rate variability diagram 24. For example, in a case where the period feed button 73b is operated, the scroll frame 72 moves to the right side, and information about the fetal heart rate or the LTV and STV in a period adjacent to the right side of the current display period (period after current timepoint and newer information than current timepoint) is displayed on the fetal heart rate diagram 23 and the heart rate variability diagram 24.

On the other hand, in a case where the period return button 73a is operated, the scroll frame 72 moves to the left side, and information about the fetal heart rate or the LTV and STV in a period adjacent to the left side of the current display period (period before current timepoint and older information than current timepoint) is displayed on the fetal heart rate diagram 23 and the heart rate variability diagram 24. With a subsequent operation of the period return button 73a, the scroll frame 72 can be further moved to the left side, and information about the fetal heart rate or the LTV and STV at the position can be displayed on the fetal heart rate diagram 23 and the heart rate variability diagram 24. As described above, in a case where the period return button 73a and the period feed button 73b are used, the information about the fetal heart rate or the LTV and STV for each period surrounded by the scroll frame 72 can be displayed on the fetal heart rate diagram 23 and the heart rate variability diagram 24 in order.

As shown in Fig. 3, the period designation unit 42 that can designates a position having a further short period (designated period) within the display time axis displayed on the fetal heart rate diagram 23 is provided on the fetal heart rate diagram 23 during the playback. The short period at this time is, for example, several seconds. The period designation unit 42 is moved in the left and right direction of the screen to dispose the unit at a predetermined position within the display time axis of the fetal heart rate diagram 23. With this, it is possible to display the fetal bioelectric signal (waveform) in a period in which the period designation unit 42 is overlapped and disposed on the fetal bioelectric signal diagram 22 and display the maternal electrocardiogram signal (waveform) in the period on the maternal electrocardiogram 21. In the maternal electrocardiogram 21 and the fetal bioelectric signal diagram 22, the designated period designated by the period designation unit 42 is indicated by an area 42a and an area 42b, respectively.

The fetal bioelectric signal waveform within the designated period designated by the period designation unit 42 is displayed in the area 42b in the fetal bioelectric signal diagram 22. The fetal bioelectric signal waveform is continuously displayed including periods before and after (left and right) of this area 42b, that is, periods before and after adjacent to the designated period, and a background color is changed between the area 42b and an area other than the area 42b. Accordingly, the user can clearly distinguish a difference between the fetal bioelectric signal in the designated period and the fetal bioelectric signal in another period.

Similar to the fetal bioelectric signal diagram 22, the area 42a is also displayed in the maternal electrocardiogram 21, and the background color is changed between the area 42a and the area other than the area 42a. Therefore, the user can clearly distinguish the difference between the maternal electrocardiogram signal in the designated period and the maternal electrocardiogram signal in another period. The maternal electrocardiogram 21 and the fetal bioelectric signal diagram 22 always display information in the same time range.

The period designation unit 42 is a marker having a vertical line displayed in a width of one pixel to several pixels on the screen, and a time corresponding to this width is designated as the designated period and is indicated by the areas 42a and 42b on the maternal electrocardiogram 21 and the fetal bioelectric signal diagram 22. For example, when the width of the period designation unit 42 corresponds to one second, for example, one second is designated as the designated period. In the maternal electrocardiogram 21 and the fetal bioelectric signal diagram 22, in a case where a display setting of the time axis on the screen is, for example, 1/100 of the fetal heart rate diagram 23, a width of 100 times the width of the period designation unit 42 is surrounded by the areas 42a and 42b.

In Fig. 3, the scroll bar 71 shows information for the entire measurement time (for example, several hours), and the fetal heart rate diagram 23 shows information for a time surrounded by the scroll frame 72 (for example, 15 minutes). On the contrary, the maternal electrocardiogram 21 and the fetal bioelectric signal diagram 22 show information for a shorter time (for example, 10 seconds) than the fetal heart rate diagram 23 in an enlarged manner, and the designated period (for example, one second) designated by the period designation unit 42 is indicated by the areas 42a and 42b.

As means for displaying the fetal bioelectric signal within the designated period designated by the period designation unit 42 and the fetal bioelectric signal in another period adjacent to the designated period in an identifiable manner, the background color of the areas 42a and 42b is changed to a color different from another period. Various pieces of means such as changing a color of a line indicating the waveform of the fetal bioelectric signal in the designated period to a color different from another period in addition to being displayed by a frame line surrounding a part of the fetal bioelectric signal diagram 22 or changing a thickness of the waveform of the fetal bioelectric signal can be employed.

As described above, in the monitoring device 101 of the present embodiment, when the designated period is designated by the period designation unit 42, the fetal bioelectric signal and the maternal electrocardiogram signal in the designated period can be displayed on the display unit 12 and thus can be easily checked by the user.

A simplified icon or the like corresponding to the simple event recording button 61c or the like displayed on the fetal heart rate diagram 23 is displayed on the scroll bar 71, and the user can easily check the presence or absence of the event information on the scroll bar 71. Therefore, the user can easily recognize a correlation between the event information and the information of the fetal heart rate diagram 23.

An event display return button 75a and an event display feed button 75b are disposed on the left and right of the scroll bar 71. With operating (pressing or touching) of the event display return button 75a and the event display feed button 75b, it is possible to move the scroll frame 72 from a position where the scroll frame 72 is currently disposed to a period in which the event information at the nearest position is recorded. It is possible to instantaneously display the information about the fetal heart rate and the labor pain intensity in this period on the fetal heart rate diagram 23.

In a case where the event display feed button 75b is operated, the fetal heart rate diagram 23 is displayed with the event information disposed at the nearest position from the current scroll frame 72 in a range of the right side of the screen as the center of the display period. When the event display feed button 75b is subsequently operated, the fetal heart rate diagram 23 is displayed with the event information on the further right side of the screen as the center of the display period from the event information disposed at the center position of the current display period. Similarly, in a case where the event display return button 75a is operated, the fetal heart rate diagram 23 is displayed with the event information disposed at the nearest position from the current scroll frame 72 in a range of the left side of the screen as the center of the display period. As described above, in the monitoring device 101, the event information can be easily accessed by operating the event display return button 75a and the event display feed button 75b.

The operating unit 14 includes the event display return button 75a and the event display feed button 75b that can be operated on the display unit 12, in addition to the event input unit 41 and the period designation unit 42.

In the present embodiment, the display unit 12 is configured by the touch panel type display having both the display function and the input function (operation function), but the present invention is not limited thereto. For example, an independent operating unit may be disposed beside the display unit 12. In this case, the user can easily perform the operation while checking the display unit 12.

### [Operation of Fetal Heart Rate Variability Monitoring Device]

Next, an operation of the monitoring device 101 will be described. When the operation (measurement) of the monitoring device 101 is started, the signal acquired from the detection unit 51 and the signal input from the information acquisition unit 52 are processed by the processing unit 11, and the fetal heart rate diagram 23 and the heart rate variability diagram 24 are displayed, as shown in Fig. 3, on the display unit 12. As described above, the fetal bioelectric signal diagram 22 for displaying the waveform of the fetal bioelectric signal and the maternal electrocardiogram 21 for displaying the waveform of the maternal electrocardiogram signal, together with the fetal heart rate diagram 23 and the heart rate variability diagram 24 are displayed on the display unit 12 at the same time.

During normal measurement of the maternal electrocardiogram signal, the fetal bioelectric signal, the fetal heart rate, and the maternal heart rate, the display unit 12 displays the information processed in real time. This information is displayed along with the time axis and is updated successively with the elapse of time.

As described above, the information of the fetal heart rate diagram 23 and the heart rate variability diagram 24 is the latest information at the right end of Fig. 3. In the recording time display area 26, a point in time at the time of measurement, which is the latest information, is displayed and the information is updated with the elapse of time. The information of the maternal electrocardiogram 21 and the fetal bioelectric signal diagram 22 is updated in order from the left end side to the right end side of Fig. 3 for each unit time. When the update point reaches the right end, the point is returned to the left end and the information is updated. The maternal heart rate and the fetal heart rate corresponding to the time at the right end of the fetal heart rate diagram 23 are displayed as numerical values in the maternal heart rate digital display area 21d and the fetal heart rate digital display area 22d.

The update of the information of the maternal electrocardiogram 21 and the fetal bioelectric signal diagram 22 is not limited thereto, and the screen may be scrolled with the elapse of time.

In a case where the user operates the event input unit 41 during monitoring, the event icons of the pieces of event information corresponding to the operated event recording buttons 41a to 41f is displayed on the fetal heart rate diagram 23. The pieces of event information are stored in the storage unit 36 together with the occurrence point in time.

As described above, in the monitoring device 101 of the present embodiment, the user designates the predetermined period for recognizing the variability information in the fetal heart rate diagram 23, and the average heart rate, the STV and LTV are notified as the variability information in the predetermined period. Therefore, it is possible to easily determine a fetal condition in the predetermined period.

The STV and the LTV are calculated at time interval set in advance and the STV and the LTV are displayed as a graph as the heart rate variability diagram 24 every time the STV and the LTV are calculated. Therefore, it is possible to recognize the change in the STV and the LTV over time and determine the fetal condition more easily.

Since the user can designate the predetermined period after recognizing the change of at least one of STV and LTV over time, it is possible to determine the fetal condition more easily. Specifically, in a case where the user has doubts about the STV or LTV values calculated and displayed at time interval set in advance, the user sets the predetermined period excluding a questionable area (for example, area where noise occurs in fetal heart rate). Therefore, it is possible to acquire the appropriate STV and LTV and determine the fetal condition more easily.

The present invention is not limited to the above embodiment, and various changes can be made in a range without departing from the spirit of the present invention.

For example, in the above embodiment, the LTV and STV calculated for each predetermined time interval are displayed on the heart rate variability diagram 24, but the present invention is not limited thereto.

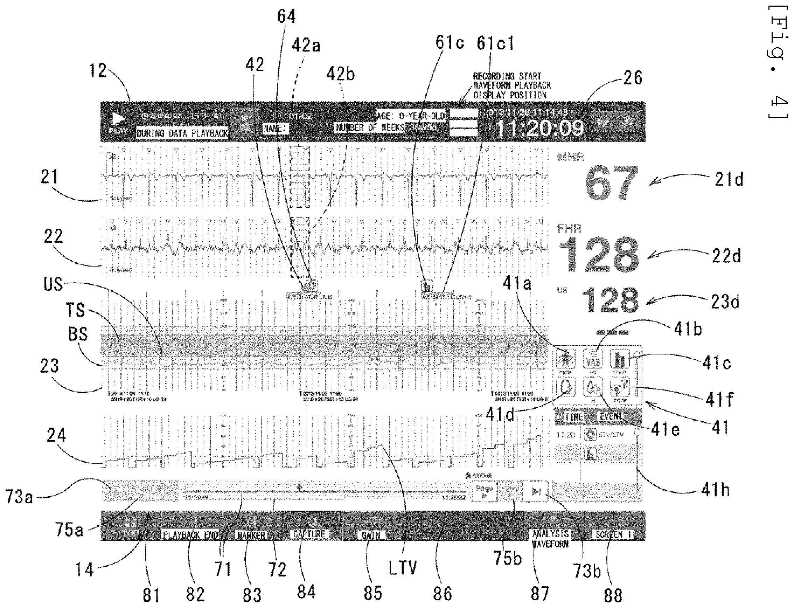

Fig. 4 is a front view of the display unit 12 of the monitoring device 101 according to a first modification example of the embodiment (in this first modification example, common elements with the embodiment are designated by the same reference numerals and description thereof is omitted, and the same applies to each of the following modification examples). In the first modification example, only the LTV calculated for each predetermined time is displayed on the heart rate variability diagram 24, as shown in Fig. 4. In this case, the LTV and STV conversion processing unit 35 needs to calculate only the LTV for each predetermined time, but the STV may also be calculated.

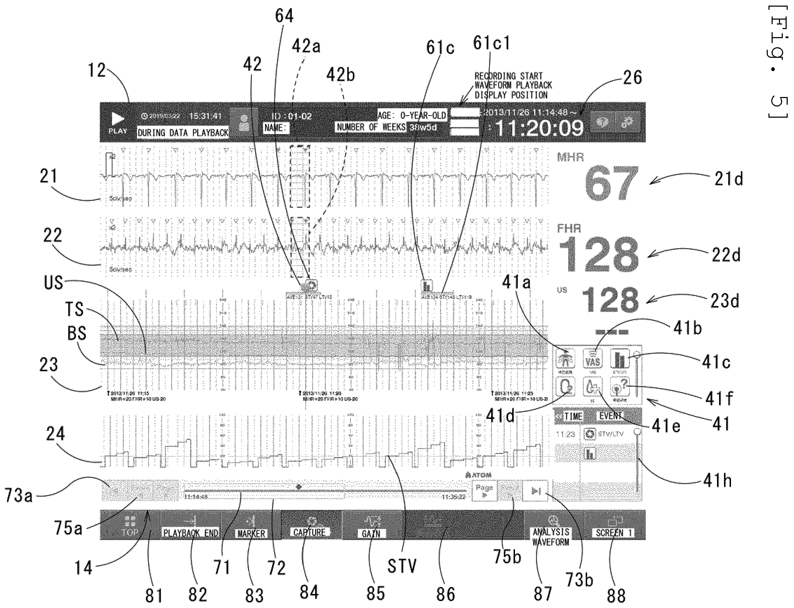

Fig. 5 is a front view of the display unit 12 of the monitoring device 101 according to a second modification example of the above embodiment. In the second modification example, only the STV calculated for each predetermined time is displayed on the heart rate variability diagram 24, as shown in Fig. 5. In this case, the LTV and STV conversion processing unit 35 needs to calculate only the STV for each predetermined time, but the LTV may also be calculated.

In the above embodiment, the STV and the LTV in the heart rate variability diagram 24 are displayed in the line graph format, but the present invention is not limited thereto.

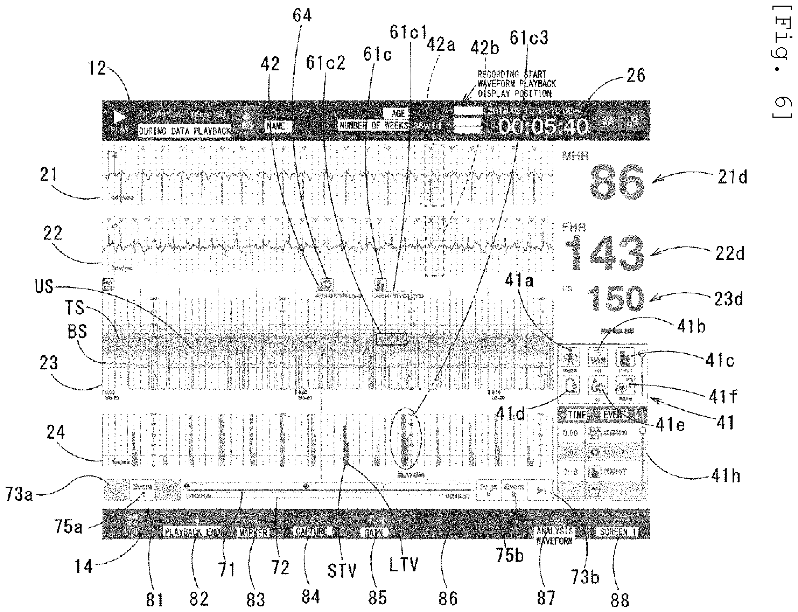

Fig. 6 is a front view of the display unit 12 of the monitoring device 101 according to a third modification example of the above embodiment. In the third modification example, the heart rate variability diagram 24 displays the STV and the LTV in a vertical bar graph format, as shown in Fig. 6. In the third modification example, in a case where the STV or the LTV calculated for each predetermined time in the heart rate variability diagram 24 overlaps with the predetermined period designated by the user, the STV and the LTV in the predetermined period are displayed in a more prominent color than the STV and the LTV in another period. For example, the STV and the LTV calculated for each predetermined time are respectively displayed in light orange and light blue, and the STV and the LTV in the predetermined period are displayed in dark orange and dark blue (reference numeral 61c3 in Fig. 6). Therefore, the user can easily recognize the STV and the LTV in the predetermined period also on the heart rate variability diagram 24, together with the numerical values of the STV and the LTV displayed below the bar 61c1 indicating the predetermined period.

Even in a case where the STV or the LTV calculated for each predetermined time in the heart rate variability diagram 24 does not overlap with the predetermined period designated by the user, the STV or the LTV in the predetermined period may be displayed in a color different from the STV or the LTV in another period.

The information indicating the predetermined period in the fetal heart rate diagram is not limited to the bar 61c1. For example, the predetermined period may be divided and displayed on the fetal heart rate diagram 23, or the color of the fetal heart rate curve or the background color of the fetal heart rate diagram in the predetermined period may be displayed in a color different from those other than the predetermined period. The marker or the like may be displayed only at the start point and the end point of the predetermined period.

In the above embodiment, the labor pain intensity is not displayed on the display unit 12, but the labor pain intensity may be displayed on the display unit 12.

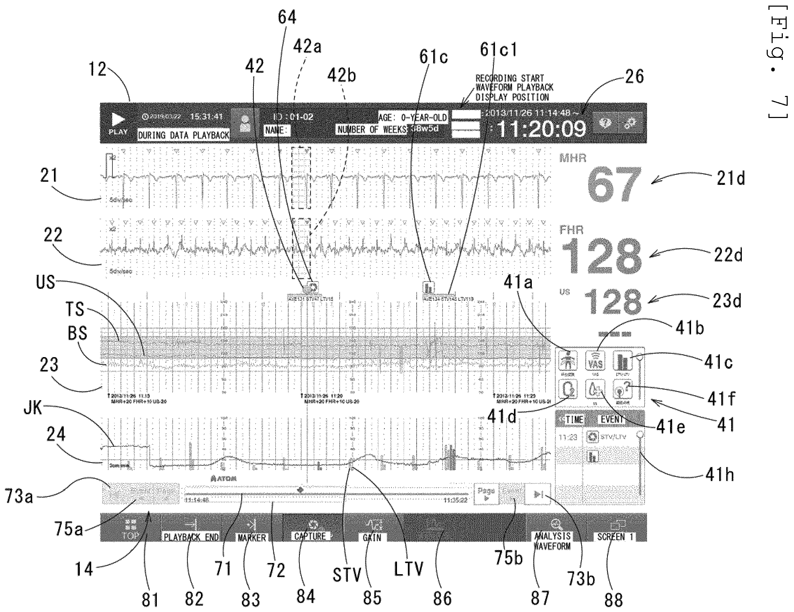

Fig. 7 is a front view of the display unit 12 of the monitoring device 101 according to a fourth modification example of the above embodiment. In the fourth modification example, similarly to the third modification example, the heart rate variability diagram 24 displays the STV and the LTV in a vertical bar graph format and also displays a labor pain intensity curve JK (refer to Fig. 7). Therefore, the labor pain intensity of the maternal body can be acquired together with the fetal heart rate, the maternal heart rate, the maternal electrocardiogram signal, the fetal bioelectric signal, and the STV and LTV, and thus the user can recognize the determination of the fetal condition in more detail.

In the above embodiment, a size of the window 41h displayed in the lower right portion of the display unit 12 may be changeable.

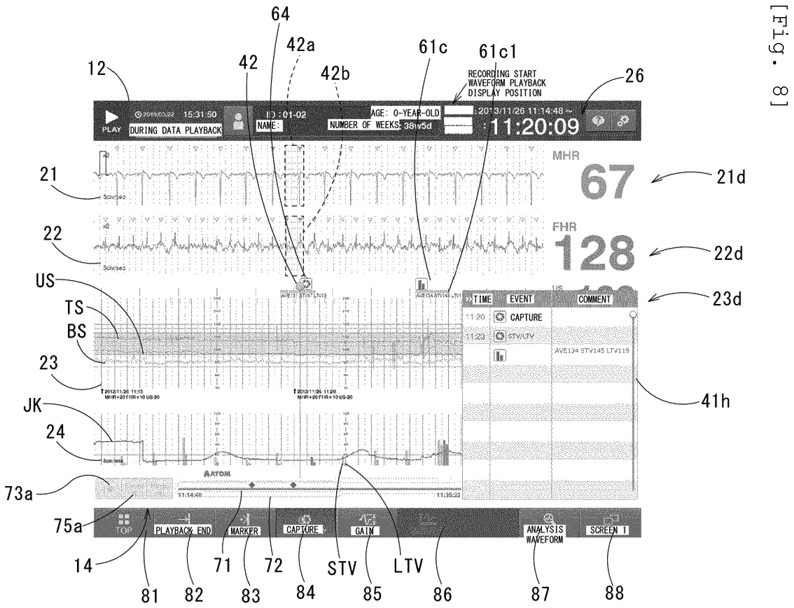

Fig. 8 is a front view of the display unit 12 of the monitoring device 101 according to a fifth modification example of the above embodiment. In the fifth modification example, the size of the window 41h can be enlarged as shown in Fig. 8. For example, when the user executes an operation of enlarging the window 41h (for example, operation of expanding interval between thumb and index finger in state of touching inside of window 41h range with thumb and index finger, so-called pinch-out), the window 41h displays the display range in an enlarged manner. Accordingly, the user can more easily recognize the various pieces of event information and a time when the event information is input.

In the above embodiment, the average fetal heart rate and STV and LTV of the fetus within the predetermined period are notified as information about the fetal heart rate variability (variability information), but the present invention is not limited thereto.

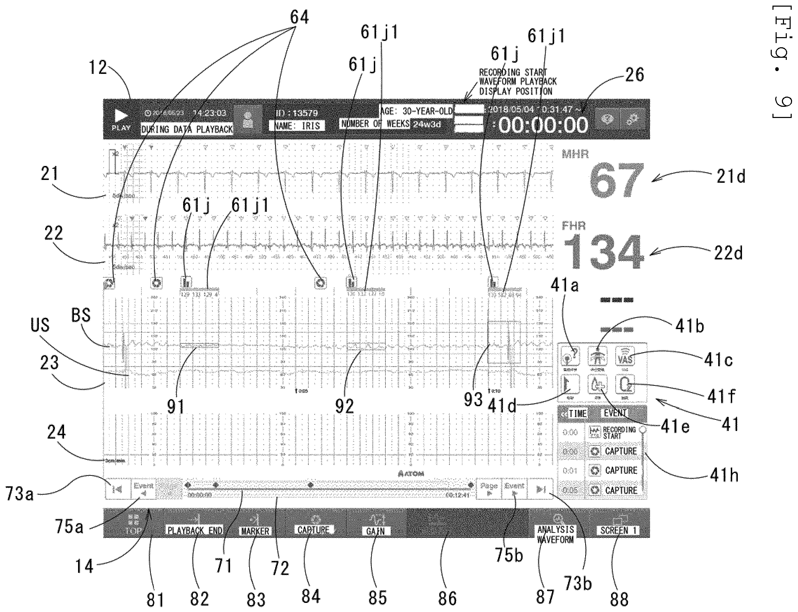

Fig. 9 is a front view of the display unit 12 of the monitoring device 101 according to a sixth modification example of the above embodiment. In the sixth modification example, the average heart rate, maximum heart rate, and minimum heart rate of the fetus and a difference between the maximum heart rate and the minimum heart rate within the predetermined period are notified, as information about the fetal heart rate variability (variability information). Specifically, frames 91, 92, and 93 surrounding the fetal heart rate curve in the predetermined period of the fetal heart rate diagram 23 are displayed as shown in Fig. 9.

Widths of the frames 91, 92, and 93 are set according to a length of the predetermined period. That is, a position at a left end of each of the frames 91, 92, and 93 corresponds to the start point of the predetermined period, and a position at a right end of each of the frames 91, 92, and 93 corresponds to the end point of the predetermined period. A height of each of the frames 91, 92, and 93 is set according to a height from a minimum value to a maximum value of the fetal heart rate in the fetal heart rate curve (difference between maximum heart rate and minimum heart rate) in the predetermined period. In Fig. 9, the height of each of the frames 91, 92, and 93 are set as the height from the minimum value to the maximum value of the fetal heart rate in the predetermined period. For example, the difference between the maximum heart rate and the minimum heart rate is "4", "10", and "94" in order from the left side of the screen for the predetermined period in which three simple event recording buttons 61j shown in Fig. 9 are disposed. Therefore, the height of the frame 91 is the smallest, and the height of the frame 93 is the largest. As described above, the height of the frame is larger as the difference between the maximum value and the minimum value of the fetal heart rate in the predetermined period is larger. Therefore, the user can recognize that the fetal heart rate variability is large simply by visually recognizing the size of the frame displayed in the fetal heart rate diagram 23 and thus it is possible to determine the fetal condition more appropriately. Since the widths of the frames 91, 92, and 93 correspond to the length of the predetermined period, the user can easily recognize the length of the predetermined period together with the magnitude of the fetal heart rate variability.

The present invention is not limited to the above frame as long as the user can recognize a difference between the magnitude of the fetal heart rate variability and the length of the predetermined period. For example, a quadrangle filled with a color, shading pattern, or the like different from another area may be displayed.

As shown in Fig. 9, the height of the frame 93 shown on the right side of the drawing is set to be smaller than the height of the fetal heart rate baseline. This is because the frame 93 is set without including the fetal heart rate determined to be noise by the separation and analysis processing unit 32. The noise determination by the separation and analysis processing unit 32 is performed by a fixed method. Accordingly, the user can visually check that the heights of the frames 91, 92, 93, that is, the variability of the fetal heart rate are calculated only by the noise-removed fetal heart rate.

In the sixth modification example, a bar 61j1 is displayed in a different aspect according to a value of the variability information. Specifically, three bars 61j1 shown in Fig. 9 are displayed in yellow-green, blue, and red in order from the left side of the drawing. The color of each bar 61j1 corresponds to the magnitude of the difference between the maximum and minimum heart rates of the fetus during the predetermined period in which each bar 61j1 is located. As described above, the bar 61j1 is displayed in a different color (aspect) according to the value of the variability information (difference between maximum and minimum heart rates of fetus). Therefore, the user can recognize the urgency of the value of the variability information according to the display aspect of the bar 61j1 and thus can reliably determine a fetal condition.

In the above embodiment and each modification example, the event recording button is disposed outside the display area of the fetal heart rate diagram 23 (right side end of screen of display unit 12), but the present invention is not limited thereto. For example, each of the event recording buttons 41a to 41f may be popped up when the user selects a desired position on the fetal heart rate diagram 23, and the predetermined period may be set when the event recording button 41c is selected from the buttons 41a to 41f. In this case, the user selects the desired position on the fetal heart rate diagram 23, and then the event recording button 41c is selected. That is, in the present invention, the selection order of the desired position on the fetal heart rate diagram 23 and the event recording button 41c does not matter.

The various aspects shown in the above modification examples can be combined individually, and the frames 91 to 93 may be displayed in the first to fifth modification examples. It is also possible to change the color of the bar according to the value of the variability information. Further, in addition to changing the color of the bar, the color of the numerical value such as the fetal heart rate displayed below the bar may be changed and displayed.

In the above embodiment, the fetal heart rate variability monitoring device 101 is configured such that the signal acquired by the delivery monitoring device is input through the information acquisition unit 52, but the present invention is not limited thereto. For example, detection means corresponding to the labor pain transducer and the ultrasonic transducer, attached to the abdomen of the maternal body, may be included. In this case, the fetal heart rate variability monitoring device 101 can also be used as the delivery monitoring device. That is, the display example of the display unit 12 can also be applied to the delivery monitoring device.

### Industrial Applicability

The present invention can be used as the fetal heart rate variability monitoring device that monitors the fetal heart rate variability.

### Reference Signs List

- 10:: device main body
- 11:: processing unit
- 12:: display unit
- 14:: operating unit
- 21:: maternal electrocardiogram
- 21d:: maternal heart rate digital display area
- 22:: fetal bioelectric signal diagram
- 22d:: fetal heart rate digital display area
- 23:: fetal heart rate diagram
- 24:: heart rate variability diagram
- 26:: recording time display area
- 31:: capturing processing unit
- 32:: separation and analysis processing unit
- 33:: maternal heart rate conversion processing unit
- 34:: fetal heart rate conversion processing unit
- 35:: LTV and STV conversion processing unit
- 36:: storage unit
- 37:: numerical processing unit
- 38:: arithmetic processing unit
- 39:: display processing unit
- 41:: event input unit (designated period input unit)
- 41a to 41f:: event recording button
- 41h:: window
- 42:: period designation unit
- 42a, 42b:: area
- 51:: detection unit
- 52:: information acquisition unit
- 61c:: simple event recording button
- 61c1, 61j1:: bar
- 71:: scroll bar
- 72:: scroll frame
- 73a:: period return button
- 73b:: period feed button
- 75a:: event display return button
- 75b:: event display feed button
- 81 to 88:: selection button
- 91, 92, 93:: frame
- 101:: fetal heart rate variability monitoring device (monitoring device)
- TS, US:: fetal heart rate curve
- BS:: maternal heart rate curve
- JK:: labor pain intensity curve

## Claims

1. A fetal heart rate variability monitoring device comprising:
display means for acquiring a fetal heart rate and displaying a fetal heart rate diagram showing the fetal heart rate over time;
calculation means for calculating variability information which is information about fetal heart rate variability from the fetal heart rate;
a designated period input unit capable of designating a predetermined period on the fetal heart rate diagram; and
notification means for issuing notification of the variability information in the predetermined period calculated by the calculation means when the predetermined period is designated by the designated period input unit.

2. The fetal heart rate variability monitoring device according to claim 1,
wherein the calculation means calculates at least one of values of STV and LTV at a time interval set in advance, and
the display means displays the value, which is successively calculated, in a graph.

3. The fetal heart rate variability monitoring device according to claim 1 or 2,
wherein the notification means causes the display means to display information indicating the predetermined period designated by the designated period input unit in correspondence with the fetal heart rate diagram.

4. The fetal heart rate variability monitoring device according to claim 3,
wherein when the predetermined period is designated by the designated period input unit, the notification means causes the display means to display the variability information in the predetermined period together with the information indicating the predetermined period near the fetal heart rate diagram.

5. The fetal heart rate variability monitoring device according to claim 4,
wherein the notification means displays at least one of the variability information and the information indicating the predetermined period in a different aspect according to a value of the variability information.

6. The fetal heart rate variability monitoring device according to any one of claims 3 to 5,
wherein the notification means displays the information indicating the predetermined period as a bar extending according to the predetermined period along a time axis of the fetal heart rate diagram.

7. The fetal heart rate variability monitoring device according to any one of claims 1 to 6,
wherein the variability information includes the information indicating the variability of the fetal heart rate,
the notification means displays an area including a fetal heart rate curve in the predetermined period of the fetal heart rate diagram, and
a size of the area in a vertical axis direction of the fetal heart rate diagram is set according to the variability information.

8. The fetal heart rate variability monitoring device according to claim 7,
wherein the notification means displays a frame surrounding the fetal heart rate curve in the predetermined period of the fetal heart rate diagram, and
a height of the frame is set according to a height from a minimum value to a maximum value of the fetal heart rate in the fetal heart rate curve in the predetermined period.

9. The fetal heart rate variability monitoring device according to any one of claims 1 to 8,
wherein the display means displays an icon for setting the predetermined period, and
when the icon and a predetermined position on the fetal heart rate diagram is selected, the designated period input unit designates the predetermined period with the predetermined position as a start point of the predetermined period.

10. The fetal heart rate variability monitoring device according to claim 9,
wherein the display means displays the icon outside the fetal heart rate diagram, and
when a movement operation from a display position of the icon to a predetermined position on the fetal heart rate diagram is detected, the designated period input unit designates the predetermined period with an end position of the movement operation as the start point of the predetermined period.
